# FASCICULE DE BREVET EUROPEEN

(11) **EP 0 668 850 B1**
(45) Date de publication et mention de la délivrance du brevet: **09.12.1998**
(21) Numéro de dépôt: 94923827.3
(22) Date de dépôt: 01.09.1994
(51) Int. Cl.: C07C 43/14, C07C 49/175

(54) **COMPOSES ALIPHATIQUES OXYGENES ET LEUR UTILISATION A TITRE D'INTERMEDIAIRES POUR LA PREPARATION DE 4-HYDROXY-2,5-DIMETHYL-3(2H)-FURANONE**
ALIPHATISCHE SAUERSTOFFVERBINDUNGEN UND IHRE VERWENDUNG ALS ZWISCHENPRODUKTE IN DER SYNTHESE VON 4-HYDROXY-2,5-DIMETHYL-3(2H)-FURANON
OXYGENATED ALIPHATIC COMPOUNDS AND THEIR USE AS INTERMEDIATES IN THE PREPARATION OF 4-HYDROXY-2,5-DIMETHYL-3(2H)-FURANONE

(30) Priorité: 14.09.1993 CH 2755/93
(43) Date de publication de la demande: 30.08.1995
(73) Titulaire: FIRMENICH SA, 1211 Genève 8 (CH)
(72) Inventeur: MIMOUN, Hubert, F-01630 Challex (FR); ZASLONA, Alexander, CH-1201 Genève (CH); LERESCHE, Jean-Paul, CH-1028 Préverenges (CH)
(74) Mandataire: Salvaterra-Garcia, Maria de Lurdes
(86) Numéro de dépôt international: IB9400261
(87) Numéro de publication internationale: WO9507876

(56) Documents cités:
- CH-A- 474 500
- TETRAHEDRON LETTERS., Juillet 1971, OXFORD GB pages 2941 - 2944 HARSH GOPAL ET AL. 'Ruthenium Tetroxide Oxidation of Alkynes. A New One Step Synthesis of alpha-Diketones' cité dans la demande
- 'METHODEN DER ORGANISCHEN CHEMIE (HOUBEN- WEYL); BAND VI/3, Sauerstoffverbindungen I' 1965 , EUGEN MüLLER , STUTTGART voir page 19 - page 20

## Description

La 4-hydroxy-2,5-diméthyl-3(2H)-furanone, mieux connue sous la dénomination commerciale de Furanéol ® [marque enregistrée de Firmenich SA, Genève] est un constituant essentiel de l'arôme d'ananas et de fraise et, à ce titre, il est employé largement dans l'industrie des arômes. Compte tenu de ses propriétés organoleptiques, il a trouvé une utilisation étendue dans de nombreuses compositions aromatisantes dans lesquelles il sert à rehausser la note fruitée et caramel caractéristique des fruits susmentionnés.

Depuis sa découverte, nombreuses ont été les synthèses suggérées pour sa préparation, parmi lesquelles il convient de citer celle qui a recours à l'emploi d'hex-3-yne-2,5-diol, comme produits de départ [voir brevet suisse n^{º} 474'500]. Ce procédé peut être représenté ainsi :

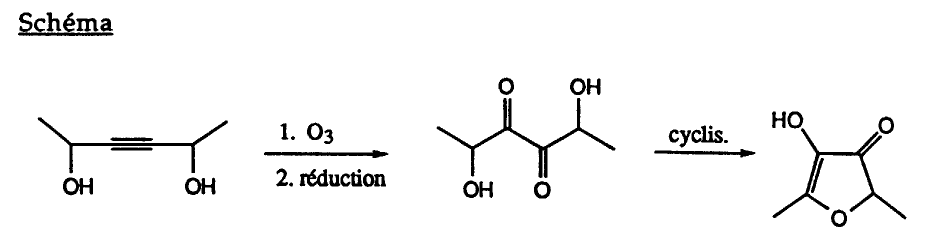

Quoique parfaitement satisfaisant au point de vue industriel, ce type de procédé est défini par des étapes critiques qui requièrent une installation spécifique et une manutention minutieuse. Il n'est pas étonnant dès lors de constater que de nombreuses équipes de recherche se soient penchées sur ce problème. Aucune méthode alternative valable cependant n'a pu s'imposer à ce jour.

### Exposé de l'invention

La présente invention entend apporter une solution nouvelle. Plus particulièrement, elle a trait à des composés aliphatiques oxygénés pouvant être employés comme intermédiaires pour la préparation du Furanéol. Les composés dont il est question ici sont les éthers ditertbutylique et diisoamylique de l'hex-3-yne-2,5-diol, ou 2,5-ditert-butyloxy-hex-3-yne et 2,5-diisoamyloxy-hex-3-yne, ainsi que le 2,5-ditert-butyloxy-hexane-3,4-dione et le 2,5-diiosamyloxy-hexane-3,4-dione. Il s'agit de composés nouveaux obtenus par un procédé original faisant également l'objet de la présente invention, lequel procédé est caractérisé par
a. la réaction de l'hex-3-yne-2,5-diol avec un composé éthylénique de formule

   (CH₃)₂ C = C (H)ₙ (CH₃)ₘ (I)

   dans laquelle l'indice n représente un nombre entier égal à 1 ou 2 et m peut prendre les valeurs zéro ou 1 et dans laquelle n+m=2, pour fournir un éther acétylénique de formule 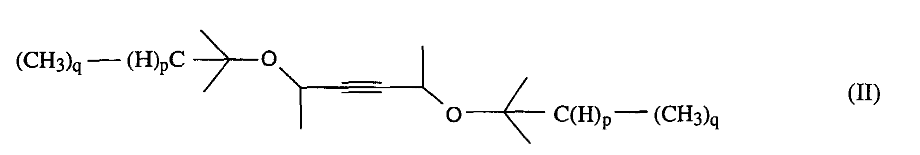 dans laquelle l'indice p représente un nombre entier égal à 2 ou 3 et q peut prendre la valeur zéro ou 1 et dans laquelle p+q=3; et
b. l'oxydation dudit éther acétylénique à l'aide de quantités catalytiques d'un système représenté par NaOCl/RuO₄ ou un peracide organique/RuO₄, pour fournir l'hexane-3,4-dione correspondante de formule 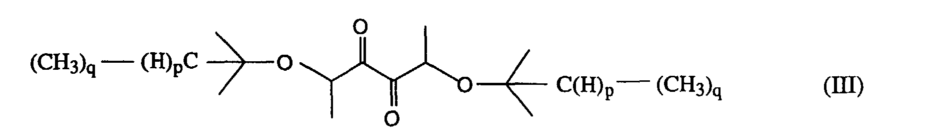 dans laquelle les indices p et q ont le sens indiqué ci-dessus.

L'hex-3-yne-2,5-diol, utilisé comme produit de départ dans le procédé de l'invention, est un produit disponible sur le marché. A titre de réactif éthylénique de formule (I), on emploie l'isobutène (n=2; m=0) ou le 2-méthyl-but-2-ène (n=m=1), ce dernier pouvant être remplacé par l'isoamylène, mélange contenant des quantités prépondérantes de 2-méthyl-but-2-ène accompagné de son isomère 2-méthyl-but-1-ène.

Cette première étape du procédé de l'invention qui consiste formellement en l'éthérification de l'hexyne-diol s'effectue de préférence en milieu anhydre et à une température inférieure à environ 80°C, de préférence comprise entre 0° et 40°C. Elle peut être conduite soit en continu, soit en batch dans un autoclave.

L'hex-3-yne-2,5-diol étant visqueux à basse température, il est préférable d'opérer en présence d'un solvant, ce qui permet également de mieux contrôler l'exothermicité de la réaction. A cette fin, des éthers tels que le méthyltert-butyl éther ou l'éther diisopropylique sont particulièrement bien adaptés. Des solvants aromatiques, tel le toluène, peuvent également être employés.

Le 2,5-ditert-butyloxy- ou le 2,5-diisoamyloxy-hex-3-yne ainsi obtenus en solution organique peuvent être employés directement sans séparation ou purification ultérieure pour l'étape suivante du procédé.

La réaction d'éthérification est également favorisée par l'action d'un catalyseur acide anhydre. Il peut s'agir d'acides protoniques, par exemple l'acide p-toluéne-sulfonique, d'acides de Lewis, tel le BF₃.Et₂O, ou encore de résines acides, telles les résines sulfoniques. Le choix particulier parmi ces réactifs sera fait sur la base de considérations économiques, de disponibilité et de protection de l'environnement. En procédant de la sorte, on a obtenu des rendements particulièrement élevés, proches des rendements quantitatifs.

La deuxième étape du procédé est caractérisée par l'oxydation de l'éther acétylénique obtenu, laquelle oxydation s'effectue à l'aide de RuO₄, ou de tout précurseur du RuO₄ pouvant donner naissance à ce dernier dans les conditions de la réaction, lequel RuO₄ est employé en quantités catalytiques. En opérant ainsi, la réaction nécessite la présence d'un agent de réoxydation du RuO₂ formé.

Ce type d'oxydation est connu en soi.

Utilisé en combinaison avec l'hypochlorite de sodium, avec des periodates ou encore avec des peracides organiques, le RuO₄ a été employé pour promouvoir bon nombre d'oxydations de substrats divers. Gopal et Gordon ont montré [Tetr. Lett. 1971 31, 2941] que le système NaOCl/RuO₄ était particulièrement bien adapté à la transformation des alcynes en α-dicétones. Appliquée pour la première fois à des substrats tels que ceux représentés par les éthers de formule (II), ce type d'oxydation offre de bons rendements en dione correspondante et est particulièrement adapté à la préparation des composés (III) de l'invention.

Selon une méthode courante, la formation initiale nécessaire de RuO₄ peut être obtenue par réaction de RuCl₃ avec l'agent oxydant, par exemple une solution de NaOCl. La proportion de RuCl₃ de départ peut varier dans une gamme de valeurs comprises entre environ 0,1 et 0,2% molaire par rapport à l'éther acétylénique de départ. La réaction est menée jusqu'à conversion complète de ce dernier en utilisant la quantité d'hypochlorite nécessaire. Celle-ci est de l'ordre de quatre équivalents par rapport à l'éther acétylénique.

La réaction d'oxydation s'effectue en dissolvant les réactifs dans un solvant organique inerte. De tels solvants sont choisis parmi les solvants chlorés, notamment le CH₂Cl₂ ou le CCl₄, ou les éthers tels le diisopropyl éther ou le méthyl-tert-butyl éther, voire des solvants aromatiques tel le toluène.

Le degré d'acidité du milieu de réaction joue également un rôle dans l'obtention de bons rendements en produit final. Nous avons observé que les rendements les meilleurs étaient obtenus lorsqu'on opère à des valeurs de pH comprises entre 5 et 9.

Comme indiqué plus haut, les nouveaux composés de l'invention sont des intermédiaires utiles pour la préparation du Furanéol ®. En effet, les éthers diones (III) peuvent être cyclisés, sous l'influence d'agents de cyclisation acides, en 4-hydroxy-2,5-diméthyl-3(2H)-furanone. Nous avons pu démontrer qu'en applicant le procédé de l'invention, l'on pouvait obtenir ce dernier avec d'excellents rendements, que l'on évitait la formation de produits secondaires, indésirables tant au point de vue de leur effet organoleptique sur le produit final qu'au point de vue de leur élimination, et que l'on évitait enfin de devoir recourir à des installations spécifiques et complexes. Un tel procédé apporte donc une amélioration certaine par rapport aux procédés connus à ce jour. Alternativement, les éthers cétoniques de formule (III) de l'invention peuvent être obtenus par réaction d'ozonolyse des éthers acétyléniques de formule (II), selon un procédé similaire aux méthodes connues en soi.

### Manières de réaliser l'invention

L'invention sera décrite plus en détail par les exemples suivants dans lesquels les températures sont indiquées en degrés centigrades.

### Exemple 1

### 2,5-Ditert-butyloxy-hex-3-yne

Un mélange de 290 g (2,54 mole) d'hex-3-yne-2,5-diol, 150 g de méthyltert-butyl éther (MTBE) et 75 g de résine sulfonique [Amberlite®, A 15] a été maintenu en agitation et chauffé à 70° de sorte à éliminer l'eau éventuellement présente à l'aide d'un séparateur.
On a ensuite refroidi le mélange à température ambiante et, après avoir remplacé le séparateur par un réfrigérant, on a introduit 100 g (1,78 mole) d'isobutylène. Après une baisse rapide à 0°/-3°, la température remonte à 30°. Une nouvelle fraction de 100 g d'isobutylène a été ajoutée au mélange et l'on procède ainsi jusqu'à atteindre une quantité totale d'isobutylène égale à 8 moles [450 g; temps d'addition: 5 h]. La solution obtenue est filtrée et la résine présente est récupérée pour une nouvelle utilisation ultérieure. La solution était ainsi prête pour l'étape suivante d'oxydation.

En opérant comme indiqué ci-dessus, mais en remplaçant l'isobutylène par un mélange d'isoamylène (85% de 2-méthyl-but-2-ène - 15% de 2-méthyl-but-1-ène), on a obtenu du 2,5-diisoamyloxy-hex-3-yne.
SM : z/e : 97, 43, 71, 79.

### Exemple 2

### 2,5-Ditert-butyloxy-hexane-3,4-dione

Un mélange constitué par 60 g de 2,5-ditert-butyloxy-hex-3-yne (0,265 mole), 60 g de méthyltert-butyl éther (MTBE) et 200 g d'eau a été maintenu à 40° sous vigoureuse agitation, en présence de 0,1 g de RuCl₃. Le pH, qui était d'environ 2,3, a été ramené à 7 avec une solution aqueuse à 30% de NaOH et une solution aqueuse à 15% de H₃PO₄.
600 g (4 équiv.) d'une solution aqueuse de NaOCl à 13,4% ont été ensuite ajoutés au mélange réactionnel, tout en maintenant le pH à env. 7 par l'introduction automatique d'une solution aqueuse d'acide phosphorique à 15%. Le déroulement de la réaction a été suivi par analyse gaz chromatographique.
Une fois atteinte la conversion complète, le mélange a été refroidi à 25° et a été décanté, puis la phase organique a été lavée avec 100 g d'eau et le RuO₄ a été séparé par décantation. Après évaporation du MTBE présent, on a récupéré la 2,5-ditert-butyloxy-hexane-3,4-dione ayant une pureté supérieure à 99% avec un rendement de 78%.

Des résultats similaires ont été obtenus en remplaçant le NaOCl par l'acide peracétique. Ce dernier est obtenu de préférence par extraction de l'acide peracétique aqueux par l'acétate d'isopropyle. La réaction s'effectue ensuite en présence de N-méthylpyrrolidone.

Le composé ainsi obtenu a servi ensuite à la préparation de 4-hydroxy-2,5-diméthyl-3(2H)-furanone par cyclisation à l'aide d'un agent de cyclisation acide.

### Exemple Comparatif

### 2,5-Ditert-bulyloxy-hexane-3,4-dione

113 g de 2,5-ditert-butyloxy-hex-3-yne, obtenu comme décrit à l'Exemple 1, et 10 g de décaline dans 280 g de méthanol ont été placés dans un bain thermostaté à -30°, puis un courant d'ozone a été introduit avec un débit de 100 l/h. Après disparition complète du produit de départ (6 h), on a dégazé sous léger courant d'air pendant 15 min, et 46 g de sulfure de diméthyle ont été ajoutés dans un intervalle de 1,5 h.
La solution résultante est maintenue au repos pendant une nuit et enfin concentrée.
On a ainsi obtenu 126 g de la dione désirée.

## Revendications

1. Ethers acétyléniques de formule 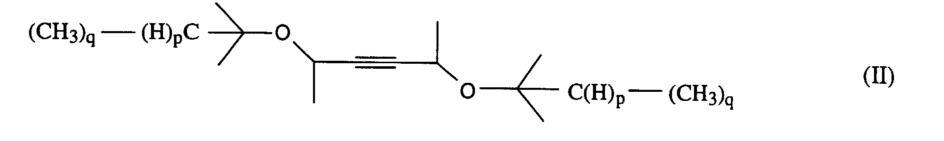 dans laquelle l'indice p représente un nombre entier égal à 2 ou 3 et q peut prendre la valeur zéro ou 1 et dans laquelle p+q=3.

2. Ethers cétoniques de formule 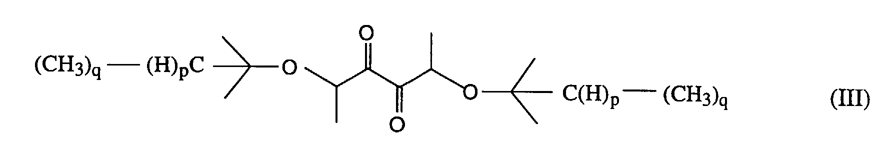 dans laquelle l'indice p représente un nombre entier égal à 2 ou 3 et q peut prendre la valeur zéro ou 1 et dans laquelle p+q=3.

3. Procédé pour la préparation d'éthers selon les revendications 1 ou 2, caractérisé en ce
a. qu'on fait réagir l'hex-3-yne-2,5-diol avec un composé éthylénique de formule
(CH₃)₂ C = C (H)ₙ (CH₃)ₘ (I)
dans laquelle l'indice n représente un nombre entier égal à 1 ou 2 et m peut prendre les valeurs zéro ou 1 et dans laquelle n+m=2, pour fournir un éther acétylénique de formule 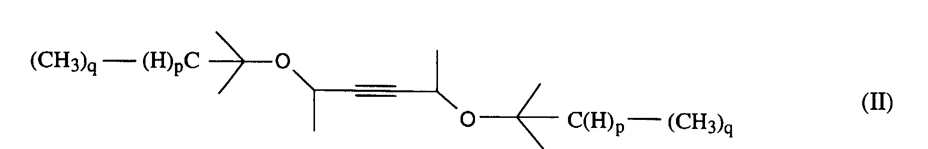 et
b. qu'on oxyde ledit éther acétylénique à l'aide de quantités catalytiques d'un système représenté par NaOCl/RuO₄ ou un peracide organique/RuO₄ pour fournir l'éther cétonique de formule 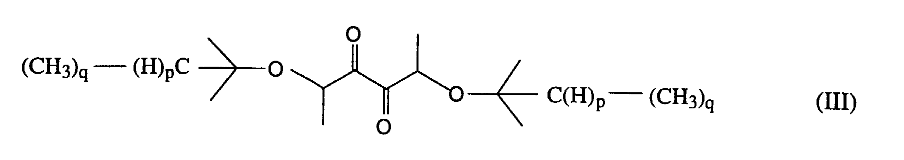 l'indice p représentant un nombre entier égal à 2 ou 3 et q pouvant prendre la valeur zéro ou 1, étant entendu que p+q=3.

4. Procédé selon la revendication 3 a., caractérisé en ce que la réaction s'effectue en milieu anhydre et à une température inférieure à 80°C.

5. Procédé selon la revendication 3, caractérisé en ce que la quantité catalytique requise de RuO₄ est obtenue in situ par réaction de NaOCl sur du RuCl₃ et que ce dernier est employé dans une proportion comprise entre environ 0,1 et 0,2% molaire par rapport à l'éther acétylénique de départ.

6. Procédé selon la revendication 5, caractérisé en ce que le NaOCl est employé dans une proportion d'environ 4 équivalents par rapport à l'éther acétylénique de départ.

7. Procédé selon l'une quelconque des revendications 3 ou 5 à 6, caractérisé en ce que l'oxydation s'effectue dans un solvant chloré ou dans un éther, de préférence le méthyl-tert-butyl éther.

8. Utilisation d'un éther cétonique selon la revendication 2 à titre de produit de départ pour la préparation de 4-hydroxy-2,5-diméthyl-3(2H)-furanone, caractérisée en ce qu'on soumet ledit éther à cyclisation.

## Claims

1. Acetylenic ethers of formula 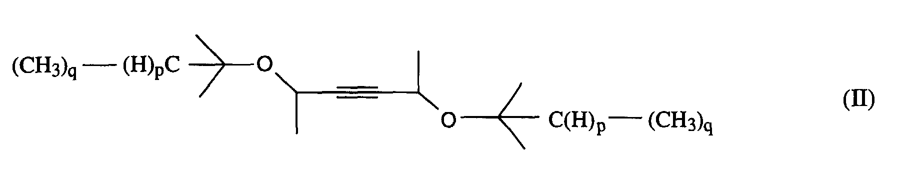 wherein index p represents an integer equal to 2 or 3 and q can take the value zero or 1 and wherein p+q=3.

2. Ketonic ethers of formula 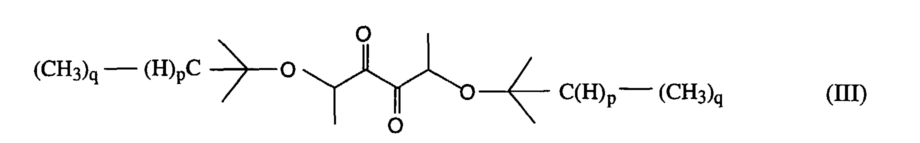 wherein index p represents an integer equal to 2 or 3 and q can take the value zero or 1 and wherein p+q=3.

3. Process for the preparation of ethers according to claims 1 or 2, characterized in that
a. hex-3-yne-2,5-diol is reacted with an ethylenic compound of formula
(CH₃)₂ C = C (H)ₙ (CH₃)ₘ (I)
wherein index n represents an integer equal to 1 or 2 and m can take the values zero or 1 and wherein n+m=2, to provide an acetylenic ether of formula 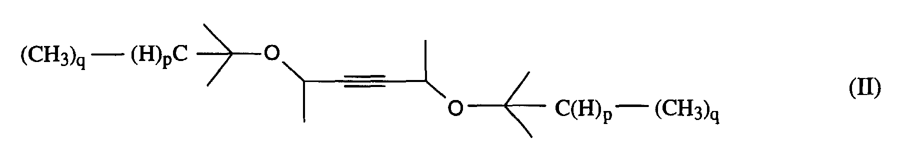 and
b. said acetylenic ether is oxidised by means of catalytic amounts of a system represented by NaOCl/RuO₄ or an organic peracid/RuO₄ to provide the ketonic ether of formula 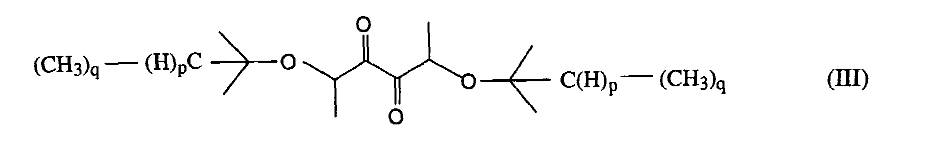 wherein index p represents an integer equal to 2 or 3 and q can take the value zero or 1, with the provision that p+q=3.

4. Process according to claim 3 a., characterized in that the reaction is carried out in an anhydrous medium and at a temperature below 80°C.

5. Process according to claim 3, characterized in that the required catalytic amount of RuO₄ is obtained in situ by reaction of NaOCl on RuCl₃ and in that the latter is used in a proportion comprised between about 0.1 and 0.2% molar, relative to the starting acetylenic ether.

6. Process according to claim 5, characterized in that NaOCl is used in a proportion of about 4 equivalents relative to the starting acetylenic ether.

7. Process according to any one of claims 3 or 5 to 6, characterized in that the oxidation is carried out in a chlorinated solvent or in an ether, preferably methyl-tert-butyl ether.

8. Use of a ketonic ether according to claim 2 as a starting product for the preparation of 4-hydroxy-2,5-dimethyl-3(2H)-furanone, characterised in that said ether is subjected to cyclization.

## Patentansprüche

1. Acetylenether mit der Formel 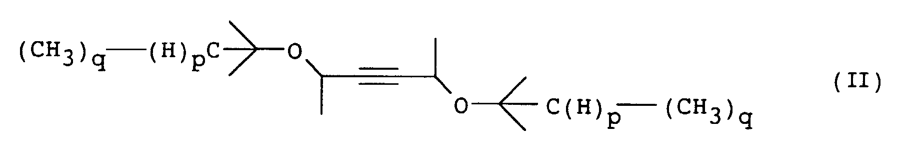 bei der der Index p für eine ganze Zahl von gleich 2 oder 3 steht und q den Wert Null oder 1 annehmen kann, und bei der gilt p+q=3.

2. Ketonether mit der Formel 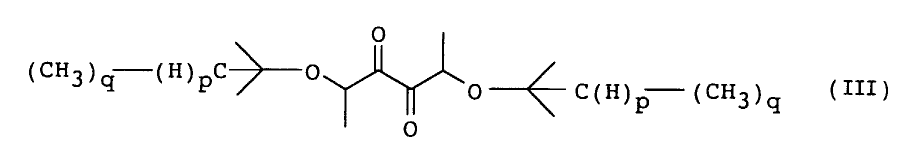 bei der der Index p für eine ganze Zahl von gleich 2 oder 3 steht und q den Wert Null oder 1 annehmen kann, und bei der gilt p+q=3.

3. Verfahren zur Herstellung von Ethern gemäß Anspruch 1 oder 2, dadurch gekennzeichnet, daß
a. Hex-3-yn-2,5-diol mit einer Ethylenverbindung mit der Formel
**(CH**_{**3**}**)**_{**2**} **C = C (H)**_{**n**} **(CH**_{**3**}**)**_{**m**} **(I)**
umgesetzt wird, bei der der Index n für eine ganze Zahl von gleich 1 oder 2 steht und m den Wert Null oder 1 annehmen kann, und bei der gilt n+m=2, um einen Acetylenether mit der Formel 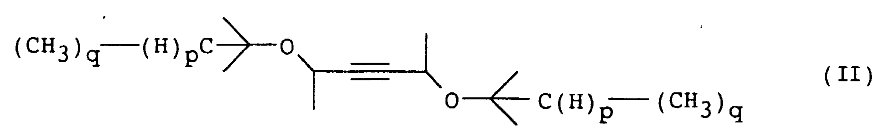 zur Verfügung zu stellen, sowie dadurch, daß
b. der Acetylenether mittels katalytischer Mengen eines durch NaOCl/RuO₄ bzw. eine organische Persäure/RuO₄ dargestellten Systems oxidiert wird, um den Ketonether mit der Formel 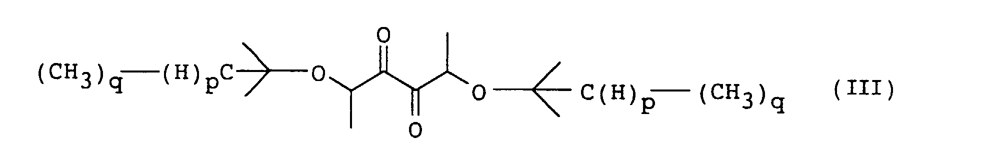 zur Verfügung zu stellen,
wobei der Index p für eine ganze Zahl von gleich 2 oder 3 steht und q den Wert Null oder 1 annehmen kann, und wobei gilt p+q=3.

4. Verfahren nach Anspruch 3a., dadurch gekennzeichnet, daß die Reaktion im wasserfreien Milieu und bei einer Temperatur von weniger als 80°C stattfindet.

5. Verfahren nach Anspruch 3, dadurch gekennzeichnet, daß die erforderliche katalytische Menge von RuO₄ in situ durch Umsetzung von NaOCl auf RuCl₃ erhalten wird, und daß das letztere in einem Anteil eingesetzt wird, der zwischen ca. 0,1 und 0,2 Mol-% in Bezug auf den Ausgangs-Acetylenether eingesetzt wird.

6. Verfahren nach Anspruch 5, dadurch gekennzeichnet, daß NaOCl in einem Anteil von ca. 4 Äquivalenten in Bezug auf den Ausgangs-Acetylenether eingesetzt wird.

7. Verfahren nach einem der Ansprüche 3 oder 5 bis 6, dadurch gekennzeichnet, daß die Oxidation in einem chlorierten Lösungsmittel oder in einem Ether, vorzugsweise Methyl-tert-Butylether, stattfindet.

8. Verwendung eines Ketonethers gemäß Anspruch 2 als Ausgangsprodukt für die Herstellung von 4-Hydroxy-2,5-dimethyl-3(2H)-furanon, dadurch gekennzeichnet, daß der Ether einer Cyclisierung unterzogen wird.
